# EUROPEAN PATENT APPLICATION

(11) **EP 4 599 869 A1**
(43) Date of publication of application: **13.08.2025**
(21) Application number: 24156740.3
(22) Date of filing: 09.02.2024
(51) Int. Cl.: A61M 5/32

(54) **NEEDLE SAFETY DEVICE FOR A MEDICAL DEVICE**

(71) Applicant: Sensile Medical AG, 4600 Olten (CH)
(72) Inventor: SOMMER, Michael, 93051 Regensburg (DE); GORSHÖFER, Andreas, 93149 Spitalhaus (DE); FORSTER, Richard, 92269 Fensterbach (DE); PFRANG, Jürgen, 93183 Kallmünz (DE); BERTUSCH, Isabel, 93053 Regensburg (DE)
(74) Representative: Bardehle Pagenberg Partnerschaft mbB Patentanwälte Rechtsanwälte

(57) **Abstract**

The present disclosure relates to a needle safety device (1) for a medical device, in particular for a medical injection device (100), the needle safety device (1) comprising: a hub portion (10) defining a longitudinal axis (11), wherein the hub portion (10) comprises a cannula (12) extending from the hub portion (10) substantially along the longitudinal axis (11), wherein the cannula (12) is configured to be moved along the longitudinal axis (11) relative to the hub portion (10); a first tubular element (20) being movably arranged on the hub portion (10) to move along the longitudinal axis (11); a second tubular element (30) being arranged on the first tubular element (20) such that the second tubular element (30) is movable along the longitudinal axis (11) together with the first tubular element (20), wherein the first tubular element (20) is at least partially enclosed by the second tubular element (30), wherein the second tubular element (30) comprises a contact surface (31) for establishing pressure contact with an injection area; wherein the first tubular element (20) and the second tubular element (30) are configured such that they allow for a rotation relative to each other; wherein the first tubular element (20) comprises a resilient member (60) and wherein the second tubular element (30) comprises a receiving member (70); wherein the resilient member (60) and the receiving member (70) are configured such that upon relative rotation of the first tubular element (20) and the second tubular element (30), at least a part of the resilient member (60) is caused to hit at least a part of the receiving member (70), preferably a hitting portion (72) of the receiving member (70), thereby generating an acoustic signal.

## Description

### 1. Technical field

The present disclosure relates to a needle safety device configured for generating an acoustic signal. The needle safe device can be used for a medical device, in particular a medical injection device. The present disclosure also relates to a respective medical device comprising the needle safety device.

### 2. Prior art

Medical devices, in particular medical injection devices, such as auto-injectors or injection pens, are known and popular among users as they provide various benefits and can be used for various applications.

An auto-injector is a device with a pre-filled syringe or vial containing a medication. It is typically designed to be automated, meaning that a user may not need to manually load the medication or set the dose. Autoinjectors are often designed to be more user-friendly and may have features like automatic needle insertion and medication delivery at the push of a button.

An injection pen is a device that typically comprises a cartridge or a vial containing the medication and a pen-like device with a needle at the tip. It usually requires manual assembly of the cartridge or vial into the pen and the setting of the desired dose before injection. The user typically needs to push a button or plunger to release the medication.

For these and further medical devices it is often necessary to ensure that no intended and/or unintended reuse of the medical injection device can take place. The reasons for this can be manifold. For example, the medical injection device in question may be intended for only one injection procedure, such as an auto-injector that injects an emergency medication like adrenaline in the event of an allergic shock. In addition, it is understood that hygiene-related reasons are also conceivable. In this regard, especially cross-contamination after injection is to be avoided, where a person is injured and contaminated by a used needle.

Various solutions, in particular needle safety devices, are known from the prior art to prevent the reuse of a medical injection device and to prevent accidental contact with a cannula of the medical injection device. In general, known needle safety devices work on the principle that a sleeve is provided which is arranged around the needle in such a way that unintentional contact with this same needle is prevented. Further, the sleeve is configured to be displaced once by contact with an injection area such that injection can take place, whereas displacement of the sleeve again after injection is prevented, exemplarily by a locking means.

For such needle safety devices, it is often necessary to provide feedback to a patient as soon as a certain operating state of the medical injection device has been reached and/or left. For example, the user should be informed whether the needle safety device is in an activated state, e.g., in a state in which it is ensured that no reuse of the same needle can take place. Another use case of such a feedback is in the case of an auto-injector that injects an emergency medication, such as adrenaline in the event of an allergic shock. In such a case, the patient must be informed when the medication has been fully dispensed. Otherwise, it cannot be ensured that the injection has been interrupted, for example due to a technical malfunction. It is understood that this could result in a significant health risk for the patient.

Various solutions are known from the prior art for providing acoustic feedback in a medical injection device. However, the specific solutions of the prior art, respectively the existing needle safety devices, have several disadvantages, some of which are presented below.

For instance, prior art medical injection devices which are configured to provide an acoustic signal, such as those described in WO 2018/082886 A1, often include assemblies with multiple parts to generate the acoustic signal. Thereby, said multiple parts are sometimes complexly arranged. Multiple parts, particularly in a complex arrangement, increase the assembly effort and/or time. In addition, the presence of multiple parts increases the risk of incorrect assembly. Furthermore, an increased number of parts also increases the manufacturing effort as more parts must be produced. Even further, the susceptibility to failure is increased, especially if the failure of a single part leads to the failure of the medical injection device and/or the assembly which serves to generate the acoustic signal.

Moreover, the parts for generating an acoustic signal within a medical injection device regularly have only the function of generating the acoustic signal. In principle, however, it would be desirable to assign more than one function to individual parts of the medical injection device. Exemplarily to reduce the total number of parts required for the medical injection device.

Thus, the proposed solutions still do not lead to optimum results and improvements are called for.

Against this background, it is an object of the present disclosure to provide a needle safety device for a medical device, in particular for a medical injection device, which addresses the aforementioned needs and that overcomes the disadvantages of the present solutions at least partially.

### 3. Summary of the invention

The above-mentioned object is at least partially achieved by the subject-matter of the independent claims. Preferred embodiments are subject of the dependent claims, and other suitable aspects of the present invention are described through the overall disclosure of the present application.

The object is achieved by a needle safety device for a medical device, in particular for a medical injection device, the needle safety device comprising: a hub portion defining a longitudinal axis, wherein the hub portion comprises a cannula extending from the hub portion substantially along the longitudinal axis, wherein the cannula is configured to be moved along the longitudinal axis relative to the hub portion; a first tubular element being movably arranged on the hub portion to move along the longitudinal axis; a second tubular element being arranged on the first tubular element such that the second tubular element is movable along the longitudinal axis together with the first tubular element, wherein the first tubular element is at least partially enclosed by the second tubular element, wherein the second tubular element comprises a contact surface for establishing pressure contact with an injection area; wherein the first tubular element and the second tubular element are configured such that they allow for a rotation relative to each other; wherein the first tubular element comprises a resilient member and wherein the second tubular element comprises a receiving member; wherein the resilient member and the receiving member are configured such that upon relative rotation of the first tubular element and the second tubular element, at least a part of the resilient member is caused to hit at least a part of the receiving member, preferably a hitting portion of the receiving member, thereby generating an acoustic signal.

In this manner, the present disclosure provides an improved needle safety device compared to known needle safety devices. In particular, the needle safety device of the present disclosure facilitates that an acoustic feedback can be provided to the user. This acoustic feedback may indicate that the safety mechanism of the need safety device is activated. This may mean that the medical device cannot be used again after usage. Hence, safety is improved in a manner convenient to the user.

The first tubular element and the second tubular element are configured such that they allow for a rotation relative to each other. This may not exclude that they rotated at least partially in the same direction, however, generating the acoustic signal, it is preferred that they rotate relative to each other.

The resilient member may refer to a component that has the ability to substantially return to its original shape or position after being bent, stretched, compressed, deflected or the like. The resilient member may comprise any kind of material, however, it is preferred that it comprises flexible and/or elastic material.

The receiving member may refer to a component that is designed to accept, hold, receive another part, component, or material and/or that is designed for being hit or the like from another part, component, or material. As detailed elsewhere herein, the receiving member may also provide for locking, preventing rotation, or the like. As described elsewhere herein, the receiving member may comprise a deflection portion, a hitting portion, a locking portion, and/or a tipping portion. One or more of these portions may be integrally formed, however, they could also be distinguished from one another as separate parts. Preferably, they are integrally formed as one piece.

At least a part of the resilient member is caused to hit at least a part of the receiving member, preferably at least a part of the resilient member is caused to hit a hitting portion of the receiving member. Hitting may be understood as contacting or the like. It is noted that the hitting may be such that an acoustic signal is generated. Thereby, it may not be sufficient that merely a gentle contact is provided. Preferably the acoustic signal is generated by impact forces due to hitting.

The needle device may be fixedly arranged with a housing of the medical device. In some examples, said fixing may be substantially permanently. However, it is also encompassed in the present disclosure that the needle safety device is releasably fixed to said housing of the medical device. In this manner, user comfort and the applicability of the needle safety device and / or the medical device can be increased.

The "hub portion" as used in the present disclosure may be an elongated hub portion. In some examples, the longitudinal axis of the hub portion may also be a longitudinal center axis of the hub portion. This may particularly be the case if the hub portion has a substantially cylindrical shape. It is understood that manufacturing tolerances may need to be considered. Thus, even when the hub portion is said to have a cylindrical shape, the shape of the hub portion may slightly deviate from a cylindrical shape. In some examples, a cross section of the hub portion may have a substantially annular shape. The hub portion may be configured such that when the needle safety device is mounted to a medical device, it may not be able to be rotated and / or moved relatively to the medical device.

The "cannula" as used in the present disclosure may additionally or alternatively be referred to as a needle or injection needle. The cannula may be hollow, so as to guide a fluid therethrough. Further, the cannula may be attached directly or indirectly to the hub portion. The cannula may be part of a cannula arrangement. For instance, the cannula arrangement may comprise a plurality of cannulas. The plurality of cannulas may be connected to one another, for instance along one direction, in particular along the longitudinal axis of the hub portion. The plurality of cannulas may partially overlap one another along the longitudinal axis, which may serve the purpose to fix the cannula arrangement.

The cannula may be able to realize a penetration of a liquid reservoir within the medical device. It is to be noted that the cannular may additionally or alternatively be moved along a different axis not parallel to the longitudinal axis. This may depend on the usage of the needle safety device. However, during ordinary use of the needle safety device, the cannula may exclusively move along the longitudinal axis. In preferred examples, the cannula maybe moved parallel to the injection direction. In preferred examples, the longitudinal axis may be parallel to the injection direction.

The first tubular element and/or the second tubular element may be a component or structure having a cylindrical or tube-like shape. In this respect, manufacturing tolerances need to be included such that deviations from a cylindrical or tube-like shape need are well encompassed in the present disclosure. In some examples, the first tubular element and/or the second tubular element may have a rounded shape. The first tubular element and/or the second tubular element may define a hollow portion, such as a chamber within their periphery. Such hollow portion may extend from one end to the opposite end, so as to accommodate one or more other parts therein. The first tubular element and the second tubular element may be referred to as a tubular arrangement in some examples. This may not mean that they are integrally formed. Preferably, the first tubular element and the second tubular element are separate pieces.

The first tubular element may comprise a guide track which slidably engages with a guide pin of the hub portion. The guide track may be configured such that the first tubular element may rotate relative to the hub portion when the guide pin moves along the guide track. This may provide for a relative rotation, for instance when pressure is provided on the contact surface of the second tubular element. This pressure may be established via an injection area.

When one or more parts are described in here to "move along the longitudinal axis", this may in some examples mean that the movement is exclusively along the longitudinal axis. However, in other examples, it does not exclude that a movement along an axis not parallel to the longitudinal axis is additionally possible. As understood, in some cases, such a movement along a different axis may be desirable. This may depend on the specific use case of the needle safety device.

The movement of the first tubular element and the second tubular element may be illustrated by the following. They may be moved from an "initial position" to an "injection position", thereby encompassing a plurality of intermediate positions between the initial position and the injection position.

The initial position and the injection position may correspond to two end positions of the first tubular element and the second tubular element. Said two end positions may be two end positions along the longitudinal axis. This may mean that beyond each of the two end positions no further movement may be possible. For instance, when they are in the injection position, there may be no further movement along the longitudinal axis in the proximal direction possible, although in some examples, this is not precluded. Further, when they are in the initial position, there may be no further movement along the longitudinal axis in the distal direction possible, although in some examples, this is not precluded.

The "injection position" may be a position in which a medication can be guided through the cannula to an injection area, such as a skin of a user. The injection position may typically mean that injection can take place.

The "initial position" may be a position in which the medication guided through the cannula when the first tubular element and the second tubular element are in the injection position, may not be guided through the cannula anymore. However, the cannula may still contain residual components of the medication, such as residual components that may not have found their way to the injection area.

The medical device maybe a medical injection device. Any kind of medical injection devices are included in the present disclosure. Exemplary, the medical injection device may be an auto-injector, an injection pen, and/or a syringe. Preferably, the medical injection device is an auto-injector. This may in particular be the case, since auto-injectors are regularly operated by non-medical personnel, e.g., in emergency situations. Thus, for these examples, the described advances of the present disclosure are particularly pronounced. However, also injection pens and other medical injection devices may significantly benefit from the advantages described in here.

It is understood that the described advantages may apply also for the following preferred embodiments.

In a preferred embodiment of the needle safety device of the present disclosure, the relative rotation is a first relative rotation and the resilient member, and the receiving member are configured such that after at least a part of the resilient member is caused to hit at least a part of the receiving member, a second relative rotation of the first tubular element and the second tubular element opposite to the first relative rotation is substantially prevented. This has the advantage that a rotation in only one direction is allowed, which may be helpful so as to provide a fail-safe arrangement for the user. The skilled person is readily aware of distinguishing between a first relative rotation (i.e., the first tubular arrangement rotates in a first direction and the second tubular arrangement rotates in a second direction) and a second relative rotation opposite to the first relative rotation (i.e., the first tubular arrangement rotates in a second direction and the second tubular arrangement rotates in a first direction)

In a preferred embodiment of the needle safety device of the present disclosure, the resilient member is integrally formed with the first tubular element, and/or the receiving member is integrally formed with the second tubular element. This has the advantage that a manufacturing is simplified, increased strength and durability is provided, the part is more cost-effective, a better fit may be provided, and it bears the possibility to reduce weight of the needle safety device. Further, less malfunctioning may be ensured.

In a preferred embodiment of the needle safety device of the present disclosure, the resilient member and the receiving member are configured such that upon relative rotation of the first tubular element and the second tubular element, the resilient member and the receiving member at least partially contact each other, preferably via a deflection portion of the receiving member, causing the resilient member to deform at least partially, optionally, the resilient member and the receiving member are configured such that upon relative rotation of the first tubular element and the second tubular element, the resilient member provides a restoring force in a direction substantially parallel to the longitudinal axis, preferably from the resilient member towards the receiving member, preferably to a deflection portion of the receiving member. This facilitates that deformation of the resilient member can be easily provided by means of the relative rotation. It is noted that the forces required to provide for the rotation may be low, whilst the deflection can still be achieved. Further, the resilient member may be configured to snap back, once a tipping point of the receiving member is passed, as described elsewhere herein. This may provide for hitting a part of the receiving member, thereby generating an acoustic signal.

In a preferred embodiment of the needle safety device of the present disclosure, the resilient member and/or a deflection portion of the receiving element has a tapered shape as seen from a side perpendicular to the axis of relative rotation. This has the advantage that the resilient member may be easily deformed. This may be the case since the cannula is usually also elongated. The term "perpendicular to the axis of relative rotation" means that it may be perpendicular to the longitudinal axis.

In a preferred embodiment of the needle safety device of the present disclosure, the at least a part of the resilient member is caused to hit at least a part of the receiving member, preferably a hitting portion of the receiving member, via an axial movement along the longitudinal axis, and/or wherein the at least a part of the resilient member is caused to hit at least a part of the receiving member, preferably a hitting portion of the receiving member, via a radial movement substantially perpendicular to the longitudinal axis.

An axial movement may be exemplarily implemented via inclined surfaces arranged at a distal or a proximal end of the first and/or second tubular element as described further below. A radial movement may be exemplarily implemented via inclined surfaces arranged at a shell surface of the first and/or second tubular element as described further below.

In a preferred embodiment of the needle safety device of the present disclosure, the resilient member and/or a deflection portion of the receiving element has an inclined surface with respect to a plane that is perpendicular to the longitudinal axis. This may further contribute to the advantages mentioned in the foregoing embodiment. Deformation of the resilient member may be simplified. As described elsewhere herein, the receiving element may comprise the deflection portion. When both, i.e., the resilient member and the deflection portion have an inclined surface, they may for instance be inclined by the same or similar angle with respect to the plane that is perpendicular to the longitudinal axis.

In a preferred embodiment of the needle safety device of the present disclosure, the inclined surface is arranged in proximity of a distal end of the first tubular element, preferably at a distal most end of the first tubular element, and/or wherein the inclined surface is arranged in proximity of a shell surface of the first tubular element. This has the advantage that the mechanism responsible for the acoustic signal may be accessed more easily. In alternative embodiments, the inclined surface may be arranged in proximity of a proximal end. When both, i.e., the resilient member and the deflection portion have an inclined surface, both of these may be arranged in proximity of a distal end of the first tubular element. The inclined surface being arranged in proximity of a shell surface of the first tubular element may mean that the inclined surface is arranged anywhere between the distal end and the proximal end of the first tubular element. It may be the case that two or more inclined surfaces are provided. One of which may be arranged in proximity of a distal end of the first tubular element and the other one may be arranged in proximity of the shell surface of the first tubular element.

In a preferred embodiment of the needle safety device of the present disclosure, the inclined surface defines an angle with respect to a plane that is perpendicular to the longitudinal axis of at least 5°, preferably at least 8°, more preferably at least 12°, more preferably at least 14°, more preferably at least 16°, and/or of at most 80°, preferably at most 70°, more preferably at most 60°, more preferably at most 50°, more preferably at most 40°, more preferably at most 30°, more preferably at most 25°, most preferably at most 22°. This contributes an improved relative rotation of the first tubular element and the second tubular element. The angle should not be too high, as this may increase a force required for movement, e.g., for rotation. The angle should not be too low, as this may reduce the acoustic signal generated. With the angles as specified in this embodiment, an optimal balance can be struck between these different and conflicting requirements.

In a preferred embodiment of the needle safety device of the present disclosure, the resilient member has an abutting surface configured to prevent the second relative rotation, wherein the abutting surface is preferably configured to contact a part of the hitting portion of the receiving member, thereby preventing the second relative rotation. This facilitates that movement can be effectively prevented such that no unintended use can be performed by the user. This may increase safety. The abutting surface may be adjacent to the inclined surface. Further, the part of the hitting portion of the receiving member that is contacted may be substantially parallel to the longitudinal axis.

In a preferred embodiment of the needle safety device of the present disclosure, the abutting surface is substantially parallel to the longitudinal axis. This allows for a relatively easy structural arrangement of the resilient member. Further, such an abutting surface may be manufactured in a simplified manner.

In a preferred embodiment of the needle safety device of the present disclosure, the receiving member, preferably a deflection portion and/or a locking portion of the receiving member, is shaped at least partially in correspondence to the resilient member. This may provide enhanced performance and functionality of the needle safety device. When these components are designed to correspond to each other, they can interact more efficiently, leading to a variety of possible effects such as the following: The resilient member may fit smoothly into the receiving member at least partially. This may prevent unwanted movements or a misfit.

In a preferred embodiment of the needle safety device of the present disclosure, the receiving member comprises a deflection portion, optionally the hitting portion, optionally a locking portion, and optionally a tipping portion, optionally, wherein upon relative rotation of the first tubular element and the second tubular element and before at least a part of the resilient member is caused to hit the hitting portion, the resilient member is configured to pass the tipping point. Optionally, the hitting portion comprises the locking portion. The deflection portion, the hitting portion, and the locking portion may be the respective portions mentioned already in previous embodiments of the present disclosure. The tipping portion may serve to initiate a change in position or orientation of a related component. This may not mean that the tipping portion is a piece separate from the receiving member, although this is not precluded. The portions mentioned in here facilitate flexibility of the movements and/or generation of the acoustic signal. It could be conceivable that the hitting portion comprises a special surface treatment such that generation of the acoustic signal is improved. In one example, the hitting portion may be the locking portion. Passing the tipping point may be understood such that upon angular movement, the tipping point of the receiving member is passed by the resilient member. This may initiate the resilient member to return into its initial shape and/or that the resilient member hits the hitting portion.

In a preferred embodiment of the needle safety device of the present disclosure after the resilient member is caused to hit the hitting portion, the first tubular element and the second tubular element are configured to rotate further in the same relative direction, causing the resilient member to engage with the locking portion. The locking portion is preferably configured to prevent a second relative rotation of the first tubular element and the second tubular element opposite to the first relative rotation is substantially prevented.

Causing the resilient member to engage with the locking portion serves several purposes, include the following: Locking can provide security and stability in the device, ensuring that the resilient member may stay in place even under physical stress, vibration, or external forces. Further, the locking mechanism can also function as a safety feature by ensuring components do not move unexpectedly.

The object of the present disclosure is also achieved by a medical device, in particular a medical injection device, comprising a needle safety device as described in the present disclosure. As understood, since the medical device comprises the needle safety device, it is understood that the features and/or advantages described with reference to the needle safety device may also apply for the medical device and vice versa.

In a preferred embodiment of the medical device of the present disclosure, the medical device is further comprising a housing which engages with the second tubular element, such that the second tubular element substantially does not rotate relative to the hub portion, wherein optionally the hub portion is affixed to the housing.

In a preferred embodiment of the medical device of the present disclosure, the medical injection device is an auto-injector or an injection pen.

### 4. Brief description of the accompanying figures

In the following, the invention will be described in more detail with reference to the following figures:
- **Fig. 1:**: shows an embodiment of the needle safety device according to the present invention in a perspective view with the second tubular element shown in a lattice structure for illustrative purpose, when the device is not activated, e.g., prior to injection.
- **Fig. 2:**: shows the embodiment of the needle safety device of Fig. 1 in a different perspective, where a part of the needle safety device is cut out for illustrative purposes.
- **Fig. 3:**: shows the embodiment of the needle safety device of Fig. 1, when the device is activated, e.g., ready for injection.
- **Fig. 4:**: shows an embodiment of a medical device according to the present disclosure, when the device is not activated.
- **Fig. 5:**: shows the embodiment of the medical device of Fig. 4, when the device is activated.

### 5. Detailed description of the figures

In the following only some possible embodiments of the invention are described in detail. However, the present invention is not limited to these, and a multitude of other embodiments are applicable without departing from the scope of the invention. The presented embodiments can be modified in a number of ways and combined with each other whenever compatible and certain features may be omitted in so far as they appear dispensable. In particular, the disclosed embodiments may be modified by combining certain features of one embodiment with one or more features of another embodiment.

Throughout the present figures and specification, the same reference numerals refer to the same elements. For the sake of clarity and conciseness, certain aspects of components or steps of certain embodiments are presented without undue detail where such detail would be apparent to those skilled in the art in light of the teachings herein and / or where such detail would obfuscate an understanding of more pertinent aspects of the embodiments.

### Definitions

The "proximal" end as used herein is merely used to illustrate one of two ends of a part. The proximal end of a part may be understood best in combination with a distal end of the same part. The terms proximal and distal are not to be understood limiting by any means. The ends could additionally or alternatively be referred to as first and second end or second and first end, respectively.

The distal end of a part may usually be an end that is further away from a center point of the medical device compared to the proximal end of the part. Further, the distal end of a part may usually be an end that is closer to an injection area compared to the proximal end of the part when the medical device is used according to its ordinary use.

Same applies to the terms "proximal direction" and "distal direction". That is, these terms could additionally or alternatively be referred to as first and second direction or second and first direction. The proximal direction may be a direction from a distal end to a proximal end. The distal direction may be a direction from a proximal end to a distal end.

Unless otherwise stated, the term "substantial" or "substantially" as used in the present context may be understood to a great or significant extent or for the most part or essentially. In particular, manufacturing tolerances are included by this term.

### Description of preferred embodiments

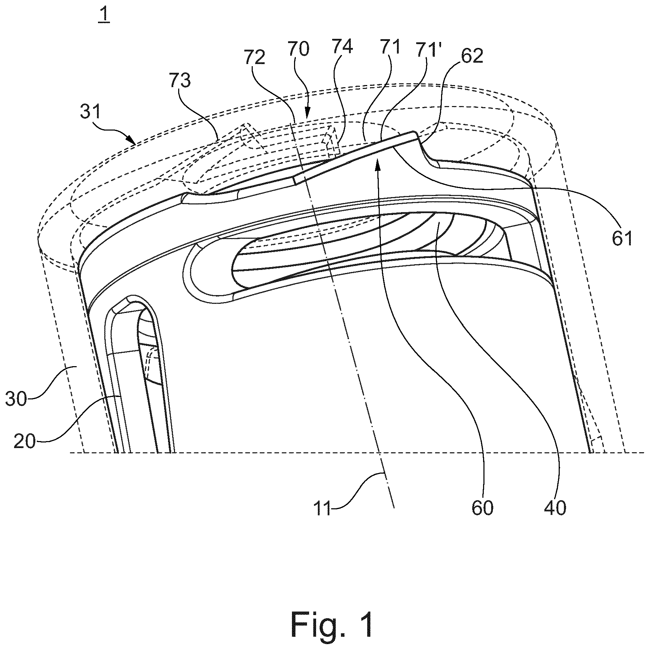

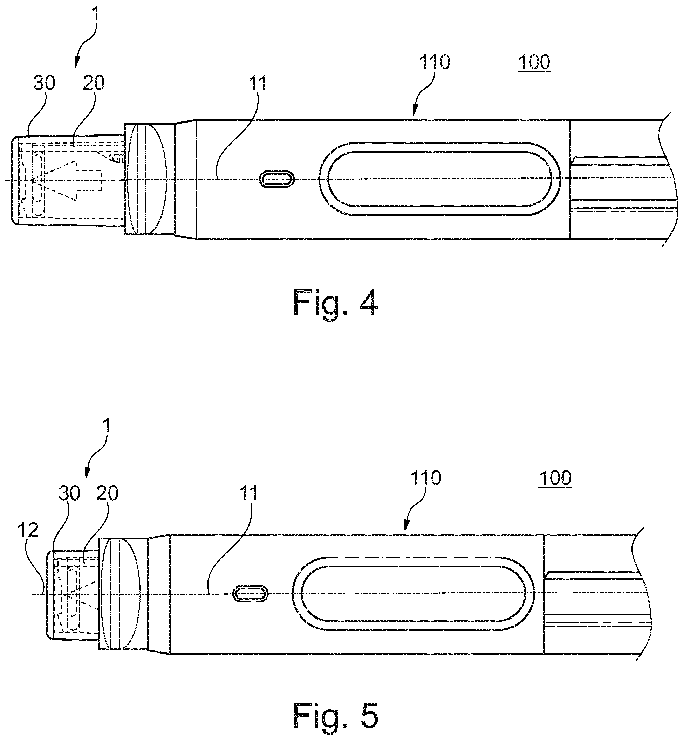

**Figs. 1 to 3** show the needle safety device 1 according to the present disclosure and/or parts thereof. The needle safety device 1 may be for a medical device, in particular for a medical injection device 100 (as can be gathered from Figs. 4 and 5).

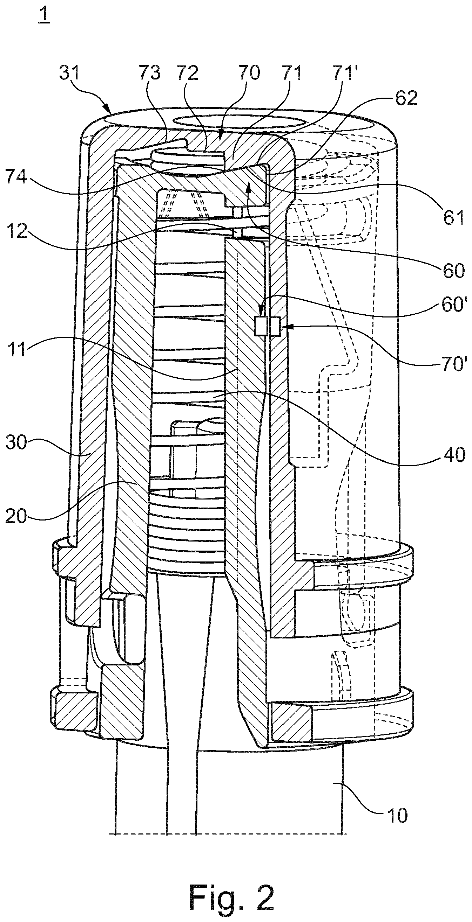

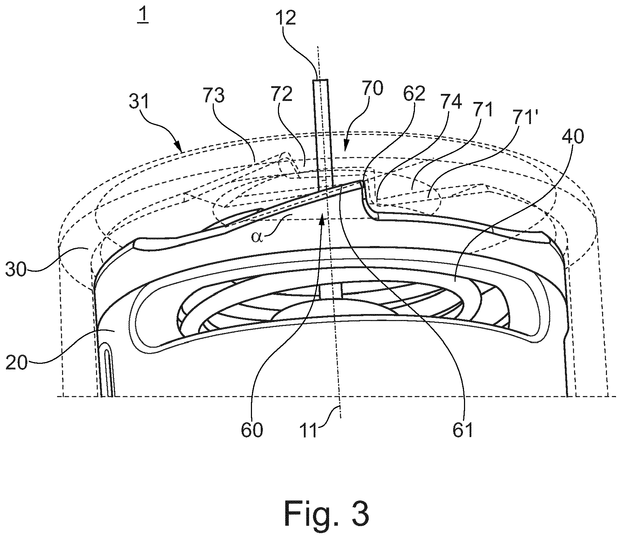

It is noted that the second tubular element 30 is shown in a lattice structure for illustrative purpose only and not with the intention to limit the scope of protection. Further, in Fig. 2, a part of the first tubular element 20 and second tubular element 30 is cut out such that the inside thereof and in particular the resilient member 60 and receiving member 70 can be seen better. Figs. 1 and 2 show the device 1 when not activated, Fig. 3 shows the device 1 when activated.

The needle safety device 1 comprises a hub portion 10 defining a longitudinal axis 11, wherein the hub portion 10 comprises a cannula 12 extending from the hub portion 10 substantially along the longitudinal axis 11. The cannula 12 is configured to be moved along the longitudinal axis 11 relative to the hub portion 10. The device 1 comprises a first tubular element 20 being movably arranged on the hub portion 10 to move along the longitudinal axis 11 and a second tubular element 30 being arranged on the first tubular element 20 such that the second tubular element 30 is movable along the longitudinal axis 11 together with the first tubular element 20. The first tubular element 20 is at least partially enclosed by the second tubular element 30 and the second tubular element 30 comprises a contact surface 31 for establishing pressure contact with an injection area, such as a part of the body of a user or another person. The first tubular element 20 and the second tubular element 30 are configured such that they allow for a rotation relative to each other.

The first tubular element 20 comprises a resilient member 60 and the second tubular element 30 comprises a receiving member 70. The resilient member 60 and the receiving member 70 are configured such that upon relative rotation of the first tubular element 20 and the second tubular element 30, at least a part of the resilient member 60 is caused to hit at least a part of the receiving member 70, preferably a hitting portion 72 of the receiving member 70 (as best seen when comparing Fig. 1 and 3), thereby generating an acoustic signal.

The generation of the acoustic signal may be illustrated as follows: The resilient member 60 may function as a bending beam, which is being deformed via the deflection portion 71 of the receiving member 70. This may introduce tension into the resilient member 60. Due to a further relative rotation, the resilient member 60 may pass over a tipping point 74, which may be the tip of sawtooth (Figs. 1 to 3). Subsequently, the resilient member 60 may be relaxed, for instance abruptly relaxed. Further, the generation of the acoustic signal may be explained by way of the following without the intention to limit the scope of protection: When two surfaces hit each other, their impact generates vibrations which travel through the medium around them as waves. These waves, when they reach a receiver such as our ears or a microphone, are perceived as sound. The intensity, frequency, and characteristics of this signal may vary depending on factors such as the type of surfaces involved, the speed and angle of the impact, and the surrounding medium.

As can be seen in Figs. 1 to 3, the resilient member 60 can be integrally formed with the first tubular element 20, and/or the receiving member 70 can be integrally formed with the second tubular element 30.

As can be further gathered, the resilient member 60 and the receiving member 70 can be configured such that upon relative rotation, the resilient member 60 and the receiving member 70 at least partially contact each other. This contact may take place via the deflection portion 71 of the receiving member 70. This may cause the resilient member 60 to deform at least partially. Further, upon relative rotation, the resilient member 60 provides a restoring force in a direction substantially parallel to the longitudinal axis 11, preferably from the resilient member 60 towards the receiving member 70, preferably to a deflection portion 71 of the receiving member.

The relative rotation can be a first relative rotation and the resilient member 60 and the receiving member 70 can be configured such that after at least a part of the resilient member 60 is caused to hit at least a part of the receiving member 70, a second relative rotation of the first tubular element 20 and the second tubular element 30 opposite to the first relative rotation is substantially prevented.

The resilient member 60 and/or the deflection portion 71 of the receiving element 70 can have a tapered shape as seen from a side perpendicular to the axis of relative rotation. Further, the resilient member 60 can have an inclined surface 61. Moreover, the deflection portion 71 can have an inclined surface 71'.

The resilient member 60 can also have an abutting surface 62 configured to prevent the second relative rotation. For this, the abutting surface 62 may contact a part of the hitting portion 72 (as best seen in Fig. 3), thereby preventing the second relative rotation. The abutting surface 62 is substantially parallel to the longitudinal axis 11.

The receiving member 70, preferably the deflection portion 71 and/or the locking portion 73, is shaped at least partially in correspondence to the resilient member 60. This has the advantage that device works more efficiently and/or effectively because the components are designed to work in harmony. When components are designed to fit together properly, they can absorb stress or wear more evenly, potentially increasing the lifespan of the device. In the case of the deflection portion 71, the performance could be optimized for better absorbance of impact or force. In the case of the locking portion 73, it could provide a secure connection, ensuring that the resilient member 60 may stay firmly in place.

Correspondingly shaped parts can be easier to assemble and may require fewer additional components, saving time and potentially reducing costs.

Upon relative rotation of the first tubular element 20 and the second tubular element 30 and before at least a part of the resilient member 60 is caused to hit the hitting portion 72, the resilient member 60 is configured to pass the tipping point 74 (as best seen when comparing Fig. 1 and 3).

Further, after the resilient member 60 hits the hitting portion 72 (as best seen in Fig. 3), the first tubular element 20 and the second tubular element 30 may be configured to rotate further in the same relative direction. This may cause the resilient member 60 to engage with the locking portion 73. This allows for better control, as you can lock or unlock the movement as per the requirements. The locking function can protect components by preventing unwanted motion or alterations in position that might lead to wear-and-tear or damage.

As best seen in Fig. 3, the device 1 is further comprising a spring element 40 which biases the second tubular element 30 in a distal direction relative to the hub portion 10.

Further, Fig. 3 shows that the inclined surface 61, 71' defines an angle α (indicated with the help of two dashed lines for surface 61, which applies similarly to surface 71') with respect to a plane that is perpendicular to the longitudinal axis 11 of at least 5° and/or of at most 80° as described elsewhere in here.

As can be seen in Figs. 1 to 3, the resilient member 60 and/or the receiving member 70, preferably a part of the resilient member 60 and/or a part of the receiving member 70, are arranged on an axial end of the first tubular element 20 and/or an axial end of the second tubular element 30.

Thereby, the at least a part of the resilient member 60 may be caused to hit at least a part of the receiving member 70, preferably a hitting portion 72 of the receiving member 70, via an axial movement along the longitudinal axis 11.

Moreover, Fig. 2 exemplarily shows that the resilient member 60' and/or the receiving member 70' may be arranged on a shell surface of the first tubular element 20 and/or a shell surface of the second tubular element 30, respectively, along the longitudinal axis 11. The shell surface of the first tubular element 20 and/or the shell surface of the second tubular element 30 may be referred to as a peripheral surface of the first tubular element 20 and/or a peripheral surface of the second tubular element 30, respectively.

Thereby, the at least a part of the resilient member 60' may be caused to hit at least a part of the receiving member 70', preferably a hitting portion 72 of the receiving member 70', via a radial movement. This movement may be substantially perpendicular to the longitudinal axis 11.

It is noted that the same features and benefits described with reference to the resilient member 60 and/or to the receiving member 70 may apply to the resilient member 60' and/or the receiving member 70' (arranged on said shell surface). Moreover, reference numeral 60' merely serves to indicate a location of arrangement and does not limit the resilient member with reference numeral 60 any further. Further, it is noted that reference numeral 70' merely serves to indicate a location of arrangement and does not limit the receiving member with reference numeral 70 any further.

The receiving member 70, preferably the deflection portion 71, the hitting portion 72, and the locking portion 73 spans an angular section about the axis of rotation of the first tubular element 20 and the second tubular element 30 of at least of at least 5°, preferably at least 10°, more preferably at least 20°, more preferably at least 30°, and/or of at most 180°, preferably at most 90°, more preferably at most 80°, more preferably at most 70°, more preferably at most 60°, most preferably at most 50°.

The angular section should not be too large as this necessitates more material for the parts in order to generate the acoustic signal. Further, the angle should not be too small, as this may require a rather steep angle of the deflection portion or the like, leading to a greater resistance of relative movement. With the angles as specified in this embodiment, an optimal balance can be struck between these different and conflicting requirements.

The resilient member 60 can comprise one or more of the following materials: a polymer, a metal. The resilient member 60 is preferably an injection molded member. The receiving member 70 comprises one or more of the following materials: a polymer, a metal. The receiving member 70 is preferably an injection molded member. The receiving member 70 preferably has a greater stiffness compared to the resilient member 60. This may allow that the resilient member 60 can deform more easily.

As can be gathered from Figs. 1 to 3, the deflection portion 71 and the locking portion 73 each have a tapered shape as seen from a side perpendicular to the axis of relative rotation, preferably a shape of one or more saw teeth. Further, the hitting portion 72 has a substantially flattened shape as seen from a side perpendicular to the axis of relative rotation.

The first tubular element 20 may comprise a guide track which slidably engages with a guide pin of the hub portion 10. The guide track may be configured such that the first tubular element 20 rotates relative to the hub portion 10 when the guide pin moves along the guide track. The guide track may comprise a final location configured to retain the guide pin.

The needle safety device 1 as described in here has the benefit that it may serve to prevent the reuse of a medical device, in particular a medical injection device 100. This may be achieved by way of the guide pin of the hub portion 10 described in the foregoing. Upon rotation of the first tubular element 20 relative to the hub portion 10, i.e., when the needle safety device 1 goes from the not activated to the activated state, the guide pin may substantially move along the guide track, wherein the guide track comprises a final location configured to retain the guide pin. This may mean that the guide pin is prevented from returning into other portions of the guide track.

**Fig. 4 and 5** show a medical device, in particular a medical injection device 100 according to the present disclosure which comprises the needle safety device 1 as described above. Fig. 4 shoes the not activated state. Fig. 5 shows the activated state. The medical injection device 100 comprises a housing 110 which engages with the second tubular element 30, such that the second tubular element 30 substantially does not rotate relative to the hub portion 10. Optionally, the hub portion 10 (as shown in Fig. 2) is affixed to the housing 110. Said fixing between the hub portion 10 and the housing 110 may be substantially permanently. However, the hub portion 10 may alternatively be releasably fixed to the housing 110. In this manner, user comfort and the applicability of the needle safety device 1 and/or the medical device may be increased. The medical injection device 100 can be an auto-injector or an injection pen or the like.

It is noted that any one or more of the embodiments described herein and/or examples may be combined with further aspects as described herein and details of the embodiments and/or examples may also be omitted, as will be understood by the skilled person. The scope of protection is determined by the claims and is not limited by the embodiments and/or examples disclosed in the above figures.

### List of reference signs

- 1: needle safety device
- 10: hub portion
- 11: longitudinal axis
- 12: cannula
- 20: first tubular element
- 30: second tubular element
- 31: contact surface of the second tubular element
- 40: spring element
- 60: resilient member
- 60': resilient member
- 61: inclined surface of the resilient member
- 62: abutting surface of the resilient member
- 70: receiving member
- 70': receiving member
- 71: deflection portion of the receiving member
- 71': inclined surface of the deflection portion of the resilient member
- 72: hitting portion of the receiving member
- 73: locking portion of the receiving member
- 74: tipping portion of the receiving member
- 100: medical injection device
- 110: housing
- α: angle

## Claims

1. A needle safety device (1) for a medical device, in particular for a medical injection device (100), the needle safety device (1) comprising:
a hub portion (10) defining a longitudinal axis (11), wherein the hub portion (10) comprises a cannula (12) extending from the hub portion (10) substantially along the longitudinal axis (11), wherein the cannula (12) is configured to be moved along the longitudinal axis (11) relative to the hub portion (10);
a first tubular element (20) being movably arranged on the hub portion (10) to move along the longitudinal axis (11);
a second tubular element (30) being arranged on the first tubular element (20) such that the second tubular element (30) is movable along the longitudinal axis (11) together with the first tubular element (20), wherein the first tubular element (20) is at least partially enclosed by the second tubular element (30), wherein the second tubular element (30) comprises a contact surface (31) for establishing pressure contact with an injection area;
wherein the first tubular element (20) and the second tubular element (30) are configured such that they allow for a rotation relative to each other;
wherein the first tubular element (20) comprises a resilient member (60) and wherein the second tubular element (30) comprises a receiving member (70);
wherein the resilient member (60) and the receiving member (70) are configured such that upon relative rotation of the first tubular element (20) and the second tubular element (30), at least a part of the resilient member (60) is caused to hit at least a part of the receiving member (70), preferably a hitting portion (72) of the receiving member (70), thereby generating an acoustic signal.

2. The needle safety device (1) according to the preceding claim, wherein the relative rotation is a first relative rotation and wherein the resilient member (60) and the receiving member (70) are configured such that after at least a part of the resilient member (60) is caused to hit at least a part of the receiving member (70), a second relative rotation of the first tubular element (20) and the second tubular element (30) opposite to the first relative rotation is substantially prevented.

3. The needle safety device (1) according to any one of the preceding claims, wherein the resilient member (60) is integrally formed with the first tubular element (20), and/or wherein the receiving member (70) is integrally formed with the second tubular element (30).

4. The needle safety device (1) according to any one of the preceding claims, wherein the resilient member (60) and the receiving member (70) are configured such that upon relative rotation of the first tubular element (20) and the second tubular element (30), the resilient member (60) and the receiving member (70) at least partially contact each other, preferably via a deflection portion (71) of the receiving member (70), causing the resilient member (60) to deform at least partially,
optionally, wherein the resilient member (60) and the receiving member (70) are configured such that upon relative rotation of the first tubular element (20) and the second tubular element (30), the resilient member (60) provides a restoring force in a direction substantially parallel to the longitudinal axis (11), preferably from the resilient member (60) towards the receiving member (70), preferably to a deflection portion (71) of the receiving member (70).

5. The needle safety device (1) according to any one of the preceding claims, wherein the resilient member (60) and/or a deflection portion (71) of the receiving element (70) has a tapered shape as seen from a side perpendicular to the axis of relative rotation.

6. The needle safety device (1) according to any one of the preceding claims, wherein the at least a part of the resilient member (60) is caused to hit at least a part of the receiving member (70), preferably a hitting portion (72) of the receiving member (70), via an axial movement along the longitudinal axis (11),
and/or wherein the at least a part of the resilient member (60') is caused to hit at least a part of the receiving member (70'), preferably a hitting portion (72) of the receiving member (70'), via a radial movement substantially perpendicular to the longitudinal axis (11).

7. The needle safety device (1) according to any one of the preceding claims,
wherein the resilient member (60) and/or a deflection portion (71) of the receiving element (70) has an inclined surface (61, 71') with respect to a plane that is perpendicular to the longitudinal axis (11).

8. The needle safety device (1) according to claim 7, wherein the inclined surface (61, 71') is arranged in proximity of a distal end of the first tubular element (20), preferably at a distal most end of the first tubular element (20), and/or
wherein the inclined surface (61, 71') is arranged in proximity of a shell surface of the first tubular element (20).

9. The needle safety device (1) according to any one of the preceding claims if dependent on claim 2, wherein the resilient member (60) has an abutting surface (62) configured to prevent the second relative rotation, wherein the abutting surface (62) is preferably configured to contact a part of the hitting portion (72) of the receiving member (70), thereby preventing the second relative rotation.

10. The needle safety device (1) according to claim 9, wherein the abutting surface (62) is substantially parallel to the longitudinal axis (11).

11. The needle safety device (1) according to any one of the preceding claims,
wherein the receiving member (70), preferably a deflection portion (71) and/or a locking portion (73) of the receiving member (70), is shaped at least partially in correspondence to the resilient member (60).

12. The needle safety device (1) according to any one of the preceding claims,
wherein the receiving member (70) comprises a deflection portion (71), optionally the hitting portion (72), optionally a locking portion (73), and optionally a tipping portion (74),
optionally, wherein upon relative rotation of the first tubular element (20) and the second tubular element (30) and before at least a part of the resilient member (60) is caused to hit the hitting portion (72), the resilient member (60) is configured to pass the tipping point (74),
optionally, wherein the hitting portion (72) comprises the locking portion (73).

13. The needle safety device (1) according to claim 12, wherein after the resilient member (60) is caused to hit the hitting portion (72), the first tubular element (20) and the second tubular element (30) are configured to rotate further in the same relative direction, causing the resilient member (60) to engage with the locking portion (73).

14. A medical device, in particular a medical injection device (100), comprising a needle safety device (1) according to any one of the preceding claims.

15. The medical device of the preceding claim, wherein the medical injection device (100) is an auto-injector or an injection pen.
